# EUROPÄISCHE PATENTSCHRIFT

(11) **EP 1 912 951 B1**
(45) Veröffentlichungstag und Bekanntmachung des Hinweises auf die Patenterteilung: **06.01.2010**
(21) Anmeldenummer: 06764180.3
(22) Anmeldetag: 17.07.2006
(51) Int. Cl.: C07D 235/18

(54) **HERSTELLUNG VON 1,7` -DIMETHYL-2` -PROPYL-2,5` -BI-1H-BENZIMIDAZOL**
PREPARATION OF 1,7` -DIMETHYL-2 `-PROPYL-2,5` -BI-1H-BENZIMIDAZOLE
PREPARATION DE 1,7` -DIMETHYL-2` -PROPYL-2,5` -BI-1H-BENZIMIDAZOL

(30) Priorität: 22.07.2005 DE 102005034279
(43) Veröffentlichungstag der Anmeldung: 23.04.2008
(73) Patentinhaber: Boehringer Ingelheim Pharma GmbH & Co. KG, 55216 Ingelheim am Rhein (DE)
(72) Erfinder: HEITGER, Helmut, 55218 Ingelheim Am Rhein (DE); RODRIGUEZ DEHLI, Juan, M., 55116 Mainz (DE); DACH, Rolf, 55435 Gau-Algesheim (DE)
(74) Vertreter: Hammann, Heinz
(86) Internationale Anmeldenummer: PCT/EP2006/064320
(87) Internationale Veröffentlichungsnummer: WO 2007/009967

(56) Entgegenhaltungen:
- RIES U J ET AL: "6-Substituted benzimidazoles as new nonpeptide angiotensin II receptor antagonists: synthesis, biological activity, and structure-activity relationships" JOURNAL OF MEDICINAL CHEMISTRY, AMERICAN CHEMICAL SOCIETY. WASHINGTON, US, Bd. 36, Nr. 25, 1993, Seiten 4040-4051, XP002135628 ISSN: 0022-2623 in der Anmeldung erwähnt
- KAMINSKI Z J: "2-CHLORO-4,6-DISUBSTITUTED-1,3,5-TRIAZINE S A NOVEL GROUP OF CONDENSING REAGENTS" TETRAHEDRON LETTERS, ELSEVIER, AMSTERDAM, NL, Bd. 26, Nr. 24, 1985, Seiten 2901-2904, XP001018670 ISSN: 0040-4039 in der Anmeldung erwähnt
- K. VENKATARAMAN, D. R. WAGLE: TETRAHEDRON LETTERS, Nr. 32, 1979, Seiten 3037-3040, XP002403117
- PHILIP A. HIPSKIN, ET AL.: JOURNAL OF ORGANIC CHEMISTRY, Bd. 60, 1995, Seiten 7033-7036, XP002403118

## Beschreibung

Die Erfindung betrifft an Position 2 substituierte Benzimidazole.

1,7'-Dimethyl-2'-propyl-2,5'-bi-1*H*-benzimidazol wird als Zwischenprodukt in der großtechnischen Synthese des Pharmawirkstoffs Telmisartan verwendet.

Ries et al, J. Med. Chem. (1993), 36(25), 4040-51 beschreiben die Herstellung von 1,7'-Dimethyl-2'-propyl-2,5'-bi-1*H*-benzimidazol durch Umsetzung von 2-Propyl-4-methyl-1*H*-benzimidazol-6-carbonsäure mit N-Methyl-o-phenylemdiamin in Gegenwart von Phosophorsäure.

WO 03/059890 beschreibt die Herstellung von 1,7'-Dimethyl-2'-propyl-2,5'-bi-1*H-*benzimidazol durch Umsetzung von 2-Propyl-4-methyl-1*H*-benzimidazol-6-carbonsäure mit N-Methyl-o-phenylen-diamin in Gegenwart von Methansulfonsäure und Phosphorpentoxid.

Kaminski beschreibt in Tetrahedron Letters 26(24):2901-2904 (1985) 2-Chlor-4,6-disubstituierte-1,3,5-triazine, die sich in der Peptidsynthese als nützliche Kupplungsreagenzien einsetzen lassen.

Gegenstand der vorliegenden Erfindung ist ein alternatives Verfahren zur Herstellung von 1,7'-Dimethyl-2'-propyl-2,5'-bi-1H-benzimidazol der Formel (I)

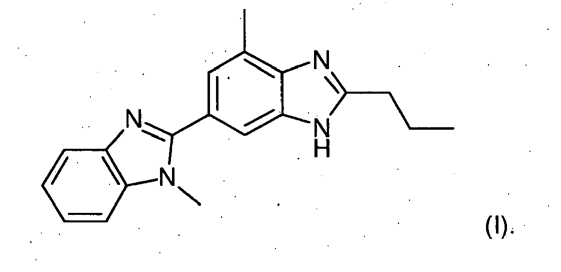

In diesem Verfahren.werden N-Methyl-o-phenylen-diamin der Formel (II) oder dessen Salze 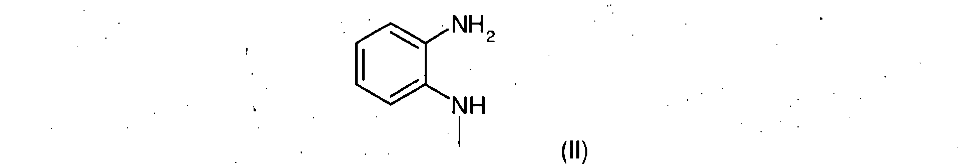 mit 2-Propyl-4-methyl-1H-benzimidazol-6-carbonsäure der Formel (III) oder deren Salzen 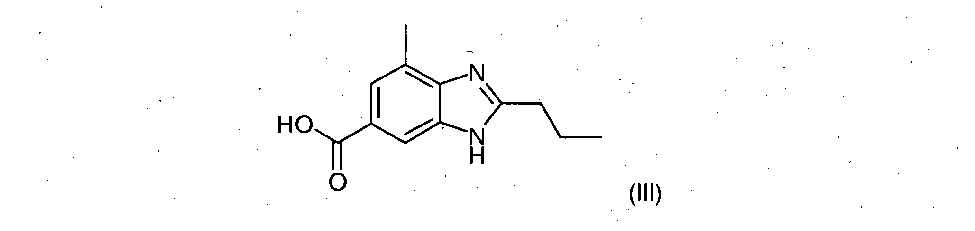 in Gegenwart eines 2-Chlor-4,6-disubstituiertes-1,3,5-triazi ns und eines tertiären Amins zu 1,7'-Dimethyl-2'-propyl-2,5'-bi-1H-benzimidazol der Formel (I) gekuppelt und zyklisiert.

Außer der Verbindung der Formel (II) können in diesem Verfahren auch dessen Salze verwendet werden. Bevorzugte Salze sind das Phosphat-, Perchlorat, Chlor- oder Bromsalz. Besonders bevorzugt ist das Phosphatsalz . Letzteres lässt sich beschreiben durch die Formel

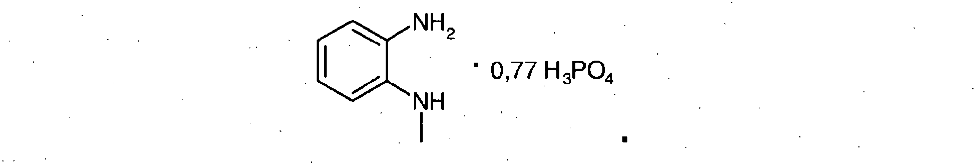

Analog können anstelle der 2-Propyl-4-methyl-1H-benzimidazol-6-carbonsäure der Formel (III) auch deren Salze verwendet werden. Bevorzugte Salze sind die Salze mit Natrium oder Kalium. Besonders bevorzugt ist die freie Carbonsäure.

Die Kupplung und Zyklisierung der beiden Ausgansverbindungen erfolgt in Gegenwart eines 1,3,5-triazins. Beispiele geeigneter Triazine sind 2,4,6-Trichlor-1,3,5-triazin, 2-Chlor-4,6-diphenoxy-1,3,5-triazin; 2-Chlor-4,6-dibenzyloxy-1,3,5-triazin; 2-Chlor-4,6-dimethoxy-1,3,5-triazin; 2,4-Dichlor-6-phenoxy-1,3,5-triazin; 2,4-Dichlor-6-benzyloxy-1,3,5-triazin oder 2,4-Dichlor-6-methoxy-1,3,5-triazin. Bevorzugt sind 2-Chlor-4,6-dialkoxy-1,3,5-triazine. Besonders bevorzugt ist 2-Chlor-4,6-dimethoxy-1,3,5-triazin.

Das Triazin wird vorher mit einem tertiären Amin aktiviert. Beispiele für geeignete tertiäre Amine sind Triethylamin, Ethyldiisopropylamin, N-methylpyrrolidin oder N-Methylmorpholin. Bevorzugt sind zyklische Amine. Besonders bevorzugt ist N-Methylmorpholin.

Als Lösungsmittel eigenen sich für dieses Verfahren polare Lösungsmittel wie N,N-Dimethylformamid, *N,N*-Dimethylacetamid, Dimethylsulfoxid, Methanol, Ethanol und 2-Propanol. Bevorzugte Lösungsmittel sind Methanol, Ethanol und 2-Propanol. Besonders bevorzugt ist Methanol.

Der erste Schritt der Reaktion (Reagenzaktivierung) kann in einem Temperaturbereich von -10°C bis 25°C, vorzugsweise bei 0°C bis 15°C und besonders bevorzugt bei 5°C durchgeführt werden. Der zweite Schritt der Reaktion (Amidbildung) kann in einem Temperaturbereich von 0°C bis 50°C, vorzugsweise bei 10°C bis 40°C und besonders bevorzugt bei 25°C durchgeführt werden. Der dritte Schritt der Reaktion (Zyklisierung) kann in einem Temperaturbereich von 20°C bis 80°C, vorzugsweise bei 40°C bis 70°C und besonders bevorzugt bei 55°C durchgeführt werden.

Ein typischer Ablauf des Verfahrens ist durch die folgenden Verfahrenschritte gekennzeichnet:
a) Aktivierung der 2-Propyl-4-methyl-1H-benzimidazol-6-carbonsäure oder deren Salz durch ein 1,3,5-Triazin und ein tertiäres Amin,
b) Kupplung der aktivierten Carbonsäure mit N-Methyl-o-phenylen-diamin oder dessen Salz zu dem entsprechenden Amid und
c) Zyklisierung des erhaltenen Amids durch Erwärmen zu 1,7'-Dimethyl-2'-propyl-2,5'-bi-1H-benzimidazol.Reinigungschritte können zwischen den Schritten (b) und (c) eingeschoben werden. 1,7'-Dimethyl-2'-propyl-2,5'-bi-1H-benzimidazol wird durch Kristallisation aufgereinigt.

Die Herstellung von 1,7'-Dimethyl-2'-propyl-2,5'-bi-1H-benzimidazol nach dem beschriebenen Verfahren gestattet Ausbeuten von mehr als 30%, vorzugsweise mehr als 60%, und hat vor allem folgende technische Vorzüge:
a) Einsatz milder Temperaturen, vorzugsweise unter 60°C,
b) Vermeidung von Phosphoroxid als Kupplungsreagenz und
c) Vermeidung von Methansulfonsäure für die Zyklisierung.

Das nach dem beschriebenen Verfahren hergestellte 1,7'-Dimethyl-2'-propyl-2,5'-bi-1H-benzimidazol der Formel (I), eignet sich zur Herstellung des Pharmawirkstoffes Telmisartan

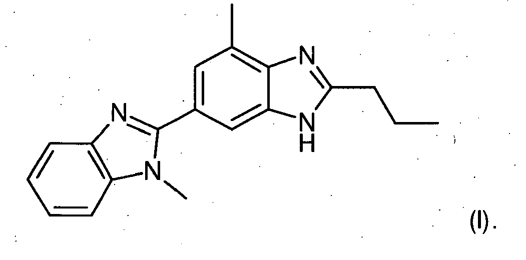

### Beispiele:

### Beipiel 1:

### Umsetzung in Gegenwart von 2-Chlor-4,6-dimethoxy-1,3,5-triazin - Variante 1

14,49 g 2-Chlor-4,6-dimethoxy-1,3,5-triazin werden in 75 mL Methanol vorgelegt und unter Rühren auf ca. 0°C abgekühlt. 9,07 mL *N*-Methylmorpholin werden während 5 Minuten bei 0°C zugetropft und noch 40 Minuten bei 0-5°C gerührt. In die klare Lösung werden 16,37 g 2-Propyl-4-methyl-1H-benzimidazol-6-carbonsäure eingetragen und dabei mit 30 mL Methanol bei 0°C nachgespült. Nach 2 Stunden bei 0°C und 2 Stunden bei 10°C werden 15 g N-Methyl-o-phenylen-diamin Phosphatsalz eingetragen und mit 7,5 mL Methanol nachgespült und die Suspension wird noch 30 Minuten bei 10°C und anschließend 2 Stunden unter Rückflusskochen gerührt. Der Ansatz wird dann über Nacht unter langsamen Rühren auf Raumtemperatur abkühlen gelassen. Der entstandene Kristallbrei wird auf 5°C abgekühlt und nach 2 Stunden langsamem Rühren abgesaugt. Die Kristalle werden mit eiskaltem Methanol gewaschen.
Ausbeute: 16,91 g (69,9% der Theorie)
HPLC: 77,9%

### Beipiel 2:

### Umsetzung in Gegenwart von 2-Chlor-4,6-dimethoxy-1,3,5-triazin - Variante 2

0,88 g 2-Chlor-4,6-dimethoxy-1,3,5-triazin in 20 mL Methanol werden vorgelegt und unter Rühren auf ca. 0°C abgekühlt. 0,55 mL *N*-Methylmorpholin werden bei 0-5°C zugetropft und noch 40 Minuten bei 0-5°C gerührt. In die klare Lösung werden 1,09 g 2-Propyl-4-methyl-1H-benzimidazol-6-carbonsäure eingetragen und dabei mit 10 mL Methanol bei 0°C nachgespült. Nach 1 Stunde bei 0°C und 1 Stunde bei Raumtemperatur werden 1,0 g N-Methyl-o-phenylen-diamin Phosphatsalz eingetragen und die Suspension wird noch über Nacht gerührt und anschließend 2 Stunden unter Rückflusskochen gerührt. Der Ansatz wird dann über Nacht unter langsamen Rühren auf Raumtemperatur abkühlen gelassen. Die Kristalle werden abgesaugt und mit Methanol gewaschen.
Ausbeute: 0,55 g (34,2% der Theorie)
HPLC: 97,7%

### Beipiel 3:

### Umsetzung in Gegenwart von 2-Chlor-4,6-dimethoxy-1,3,5-triazin - Variante 3

0,97 g 2-Chlor-4,6-dimethoxy-1,3,5-triazin in 20 mL 2-Propanol werden vorgelegt und unter Rühren auf ca. 0°C abgekühlt. 0,6 mL N-Methylmorpholin werden bei 0°C zugetropft und noch 40 Minuten bei 0-5°C gerührt. In die dicke, weiße Suspension werden 1,09 g 2-Propyl-4-methyl-1H-benzimidazol-6-carbonsäure eingetragen und dabei mit 10 mL 2-Propanol bei 0°C nachgespült. Nach 1 Stunde bei 0°C wird die Mischung auf Raumtemperatur ansteigen gelassen und weiter gerührt. Nach 2 Stunden ist das Reaktionsgemisch fast in Lösung. Dann wird der Ansatz auf 55°C erwärmt und während 75 Minuten werden 1,0 g N-Methyl-o-phenylen-diamin Phosphatsalz eingetragen und es wird 10 Stunden lang weiter gerührt. Der Ansatz wird dann abgestellt und über Nacht stehen gelassen. Die Kristalle werden abgesaugt und mit 2-Propanol gewaschen.
Ausbeute: 0,57 g (35,4% der Theorie)
HPLC: 96,8%

## Patentansprüche

1. Verfahren zur Herstellung von 1,7'-Dimethyl-2'-propyl-2,5'-bi-1H-benzimidazol der Formel (I) 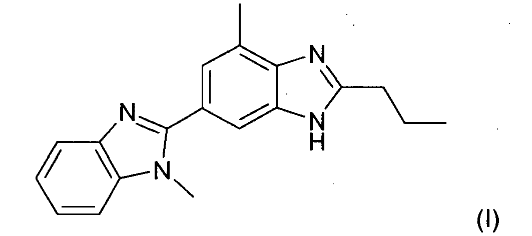 durch Umsetzung von N-Methyl-o-phenylen-diamin der Formel (II) oder dessen Salzen 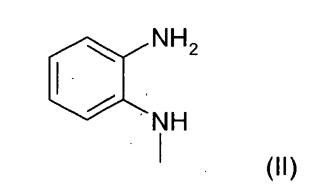 mit 2-Propyl-4-methyl-1H-benzimidazol-6-carbonsäure der Formel (III) oder deren Salzen 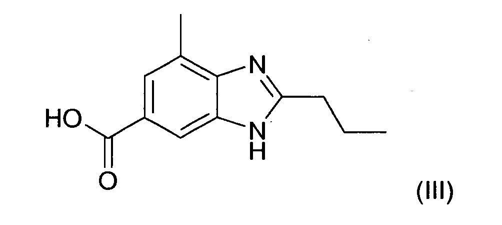 **dadurch gekennzeichnet dass** Kupplung und Zyklisierung durch ein 1,3,5-Triazin und ein tertiäres Amin erreicht wird.

2. Verfahren nach Anspruch 1 **dadurch gekennzeichnet dass** Kupplung und Zyklisierung erreicht wird durch ein 2-Chlor-4,6-disubstituiertes-1,3,5-triazin.

3. Verfahren nach Anspruch 1 **dadurch gekennzeichnet dass** Kupplung und Zyklisierung erreicht wird durch
2,4,6-Trichlor-1,3,5-triazin,
2-Chlor-4,6-diphenoxy-1,3,5-triazin,
2-Chlor-4,6-dibenzyloxy-1,3,5-triazin,
2-Chlor-4,6-dimethoxy-1,3,5-triazin,
2,4-Dichlor-6-phenoxy-1,3,5-triazin,
2,4-Dichlor-6-benzyloxy-1,3,5-triazin oder
2,4-Dichlor-6-methoxy-1,3,5-triazin.

4. Verfahren nach Anspruch 1 **dadurch gekennzeichnet dass** polare Lösungsmittel wie *N,N*-Dimethylformamid, *N,N*-Dimethylacetamid, Dimethylsulfoxid, Methanol, Ethanol oder 2-Propanol verwendet werden.

5. Verfahren nach Anspruch 1 **dadurch gekennzeichnet dass**
(a) die 2-Propyl-4-methyl-1H-benzimidazol-6-carbonsäure oder deren Salz durch ein tertiäres Amin und ein 1,3,5-Triazin aktiviert wird,
(b) die aktivierte Verbindung mit N-Methyl-o-phenylen-diamin oder dessen Salz zu einem Amid gekuppelt wird und
(c) das erhaltene Amid durch Erwärmen zu 1,7'-Dimethyl-2'-propyl-2,5'-bi-1H-benzimidazol zyklisiert wird.

6. Verfahren nach Anspruch 5 **dadurch gekennzeichnet dass**
Schritt (a) bei einer Temperatur von -10°C bis 25°C,
Schritt (b) bei einer Temperatur von 0°C bis 50°C und
Schritt (c) bei einer Temperatur von 20°C bis 80°C durchgeführt wird.

7. Verfahren nach Anspruch 1 **dadurch gekennzeichnet dass** eine Ausbeute von mehr als 30% erreicht wird.

## Claims

1. Process for preparing 1,7'-dimethyl-2'-propyl-2,5'-bi-1H-benzimidazole of formula (I) 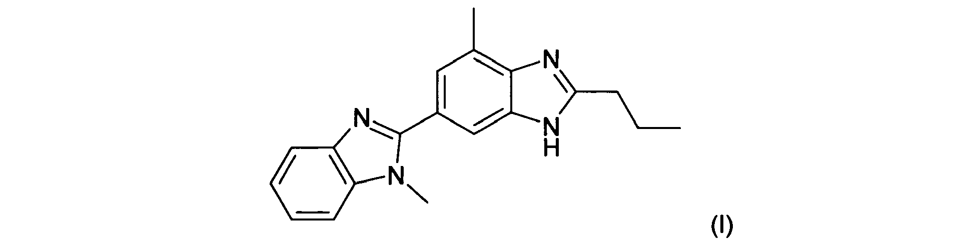 by reacting N-methyl-o-phenylene-diamine of formula (II) or the salts thereof 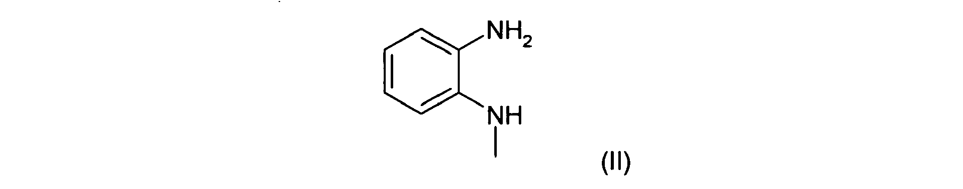 with 2-propyl-4-methyl-1H-benzimidazole-6-carboxylic acid of formula (III) or the salts thereof 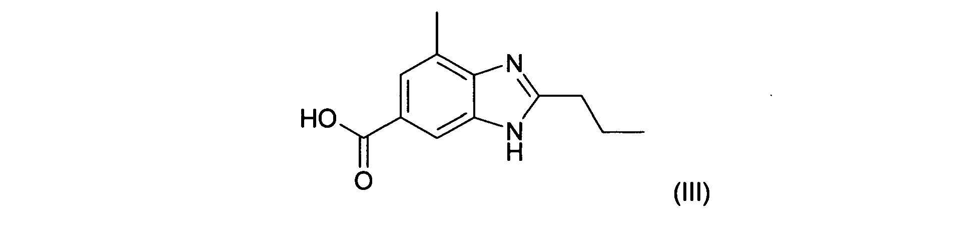 **characterised in that** coupling and cyclisation is achieved using a 1,3,5-triazine and a tertiary amine.

2. Process according to claim 1, **characterised in that** coupling and cyclisation is achieved using a 2-chloro-4,6-disubstituted-1,3,5-triazine.

3. Process according to claim 1, **characterised in that** coupling and cyclisation is achieved using
2,4,6-trichloro-1,3,5-triazine,
2-chloro-4,6-diphenoxy-1,3,5-triazine,
2-chloro-4,6-dibenzyloxy-1,3,5-triazine,
2-chloro-4,6-dimethoxy-1,3,5-triazine,
2,4-dichloro-6-phenoxy-1,3,5-triazine,
2,4-dichloro-6-benzyloxy-1,3,5-triazine or
2,4-dichloro-6-methoxy-1,3,5-triazine.

4. Process according to claim 1, **characterised in that** polar solvents such as *N,N-*dimethylformamide, *N,N*-dimethylacetamide, dimethylsulphoxide, methanol, ethanol or 2-propanol are used.

5. Process according to claim 1, **characterised in that**
(a) the 2-propyl-4-methyl-1H-benzimidazole-6-carboxylic acid or the salt thereof is activated by a tertiary amine and a 1,3,5-triazine,
(b) the activated compound is coupled with N-methyl-o-phenylene-diamine or the salt thereof to form an amide and
(c) the amide obtained is cyclised by heating to form 1,7'-dimethyl-2'-propyl-2,5'-bi-1H-benzimidazole.

6. Process according to claim 5, **characterised in that**
step (a) is carried out at a temperature of -10°C to 25°C,
step (b) is carried out at a temperature of 0°C to 50°C and
step (c) is carried out at a temperature of 20°C to 80°C.

7. Process according to claim 1, **characterised in that** a yield of more than 30% is achieved.

## Revendications

1. Procédé pour préparer le 1,7'-diméthyl-2'-propyl-2,5'-bi-1H-benzimidazole de formule (I) 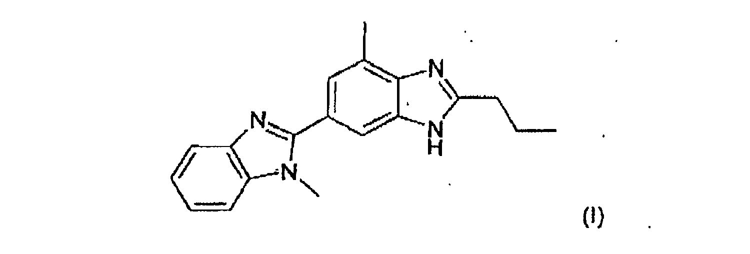 par réaction de la N-méthyl-o-phénylène-diamine de formule (II) ou de ses sels 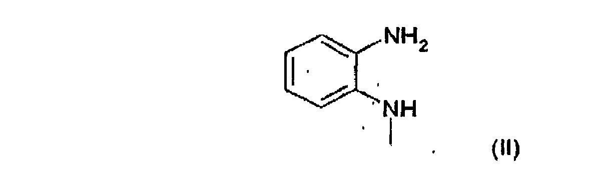 avec l'acide 2-propyl-4-méthyl-1H-benzimidazole-6-carboxylique de formule (III) ou ses sels 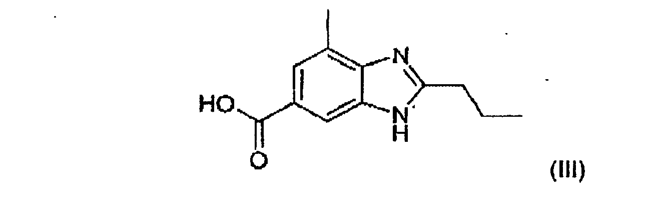 **caractérisé en ce que** le couplage et la cyclisation sont atteints par une 1,3,5-triazine et une amine tertiaire.

2. Procédé selon la revendication 1 **caractérisé en ce que** le couplage et la cyclisation sont atteints par une 2-chloro-1,3,5-triazine 4,6-disubstituée.

3. Procédé selon la revendication 1 **caractérisé en ce que** le couplage et la cyclisation sont atteints par
la 2,4,6-trichloro-1,3,5-triazine,
la 2-chloro-4,6-diphénoxy-1,3,5-triazine,
la 2-chloro-4,6-dibenzyloxy-1,3,5-triazine,
la 2-chloro-4,6-diméthoxy-1,3,5-triazine,
la 2,4-dichloro-6-phénoxy-1,3,5-triazine,
la 2,4-dichloro-6-benzyloxy-1,3,5-triazine ou
la 2,4-dichloro-6-méthoxy-1,3,5-triazine.

4. Procédé selon la revendication 1 **caractérisé en ce que** des solvants polaires comme le *N,N*-diméthylformamide, le *N,N*-diméthylacétamide, le diméthylsulfoxyde, le méthanol, l'éthanol ou le 2-propanol sont utilisés.

5. Procédé selon la revendication 1 **caractérisé en ce que**
(a) l'acide 2-propyl-4-méthyl-1H-benzimidazole-6-carboxylique ou son sel est activé par une amine tertiaire et une 1,3,5-triazine,
(b) le composé activé est couplé avec la N-méthyl-o-phénylène-diamine ou son sel en un amide et
(c) l'amide obtenu est cyclisé par chauffage en le 1,7'-diméthyl-2'-propyl-2,5'-bi-1H-benzimidazole.

6. Procédé selon la revendication 5 **caractérisé en ce que**
l'étape (a) est réalisée à une température de -10°C à 25°C,
l'étape (b) est réalisée à une température de 0°C à 50°C et
l'étape (c) est réalisée à une température de 20°C à 80°C.

7. Procédé selon la revendication 1 **caractérisé en ce qu'**un rendement supérieur à 30 % est atteint.
